# EUROPÄISCHE PATENTANMELDUNG

(11) **EP 1 589 461 A1**
(43) Veröffentlichungstag der Anmeldung: **26.10.2005**
(21) Anmeldenummer: 04009217.3
(22) Anmeldetag: 19.04.2004
(51) Int. Cl.: G06F 19/00

(54) **Elektronische Verordnung**

(71) Anmelder: Hellmann, Gunther, Dr., 91054 Erlangen (DE)
(72) Erfinder: Hellmann, Gunther, Dr., 91054 Erlangen (DE)

(57) **Zusammenfassung**

Eine elektronische Verordnung (z.B. elektronisches Rezept) besteht aus mehreren Verordnungseinheiten (z.B. Medikamente) und ggf. zusätzlichen Informationseinheiten sowie einer elektronischen Signatur, die über den zuvor genannten Teilen ermittelt wird. Dabei wird das Hash-Verfahren der Signatur einzeln auf die zuvor genannten Bestandteile angewendet und anschließend die Folge aus den zusammengesetzten Hash-Werten dem zweiten Schritt der elektronischen Signatur zugeführt. Die elektronische Verordnung wird auf einem Speichermedium (z.B: Gesundheitskarte) abgelegt oder geändert, wobei auch die Ablage auf Servern möglich ist, deren Ablageadresse über die Gesundheitskarte referenziert wird. Im Falle einer Teilbelieferung der elektronischen Verordnung wird die belieferte Verordnungseinheit durch deren Hash-Wert ersetzt.

Entsprechend der Anzahl der Verordnungseinheiten sind zusätzliche Informationseinheiten vorzusehen, die separat von der elektronischen Verordnung abgelegt werden. Die zugehörigen Hash-Werte werden in der elektronischen Verordnung integriert.

Die Berechnung der elektronischen Signatur wird um ein Hash-Verfahren ergänzt, welches auf die geeignet Folge der Hash-Werte zuvor genannter Bestandteile angewendet wird.

Die elektronische Verordnung wird außerhalb ergänzt um Zusatzinformationen, die Verfahrensdaten und deren zugehörige Signaturen aufnehmen kann.

## Beschreibung

### Stand der Technik:

Es ist üblich, elektronische Dokumente mit einer elektronischen Signatur zu versehen (Gesetz zur digitalen Signatur. Regulierungsbehörde für Telekommunikation und Post, 2001 oder Kühn, U.: Technische Grundlagen digitaler Signaturverfahren. In Hoeren, T., Schüngel M. [Hrsg.]: Rechtsfragen der digitalen Signatur. Erich Schmidt Verlag, S. 65 -91, 1999), z.B. im speziellen das elektronische Rezept (Debold & Lux: Kosten-Nutzen-Analyse Neue Versichertenkarte und Elektronisches Rezept, Endbericht, Hamburg, 18.5.2001).

Das elektronische Rezept z.B. stellt dabei eine spezielle Form dar, da es mehrere unabhängig voneinander einlösbare Bestandteile (Verordnungseinheiten) beinhaltet. Ferner sind die Verfahren zur elektronischen Signatur bekannt sowie die Zweiteilung der Verfahren in Hash-Algorithmus inkl. notwendiger Padding-Verfahren und Verschlüsselungsverfahren (symmetrische oder asymmetrische).

Der Grad der Verbindlichkeit der elektronischen Signatur, speziell der der fortgeschrittenen und qualifizierten Signatur, kann durch entsprechend personalisierte Prozessorchipkarten (Signaturkarten) erfüllt werden, indem zumindest der Teilschritt des Verschlüsselns auf dem Chipkartenprozessor der Signaturkarte ausgeführt wird (Managementpapiere des ATG, GVG Köln, 2000; http://atg.gvg-koeln.de/dokumente/21/atg-managementpapier_elrezept_stand_09-05-2001_print_copy.pdf).

Zu dem Themenkreis elektronische Verordnung sind weitere Schriften bekannt, die aber andere Aspekte betreffen:
- Offenlegungsschrift DE 101 64 407 A1
- Schriften des bit4health-Konsortiums, die unter www.dimdi.de publiziert sind.

Dabei ist die Zeit, die für die Berechnung der Verschlüsselung auf der Chipkarte erforderlich ist, maßgeblich für das gesamte elektronische Unterschriftsverfahren und somit die Erstellung der elektronischen Verordnung.

### Problem:

Der im Patentanspruch 1 angegebenen Erfindung liegt das Problem zugrunde, die Gesamtzeit, die für die Durchführung der elektronischen Unterschrift benötigt wird, zu reduzieren und eine Teilbelieferung der elektronischen Verordnung vor Missbrauch sicher zu kennzeichnen und durchzuführen.

Der im Patentanspruch 2 angegebenen Erfindung liegt das Problem zugrunde, die Signaturüberprüfung für Personen ohne Signaturkarte zu ermöglichen und die elektronische Verordnung vor Missbrauch sicher zu kennzeichnen und durchzuführen.

Der im Patentanspruch 3 angegebenen Erfindung liegt das Problem zugrunde, die Gesamtzeit, die für die Durchführung der elektronischen Unterschrift benötigt wird, zu reduzieren.

### Lösung:

Dieses Problem wird durch die im Patentanspruch 1, 2 und 3 aufgeführten Merkmale gelöst.

Durch Patentanspruch 4 wird die elektronische Verordnung für weitere Verfahrensschritte ergänzt.

### Erreichte Vorteile:

Die mit der Erfindung in Anspruch 1 erzielten Vorteile bestehen insbesondere darin, dass statt einer elektronischen Unterschrift pro Verordnungseinheit die ausgestellte elektronische Verordnung nur einmal in der Gesamtheit elektronisch signiert wird, der zeitkritische Berechnungsschritt durch den Chipkartenprozessor wird nur einmal durchgeführt, und dass gleichzeitig die Teilbelieferungsmöglichkeit (einzelner Verordnungseinheiten) bereits zum Erstellungszeitpunkt der elektronischen Verordnung gegeben ist.

Ein weiterer Vorteil für Anspruch 1 besteht darin, dass die Verordnungsinhalte, welche teilbeliefert werden, nicht rückschließbar verändert werden ohne dabei die elektronische Signatur zu verändern aber weiterhin die Überprüfung der Signatur erfolgreich zu ermöglichen. Andernfalls ergibt sich eine System immanente Missbrauchsgefahr. Beließe man die teilbelieferten Verordnungseinheiten im ursprünglichen verordneten Zustand, dann müsste die Kennzeichnung einer Teilbelieferung ergänzend erfolgen. Es wäre dann zwar gewährleistet, dass die elektronische Unterschrift immer erfolgreich geprüft werden kann. Eine ergänzende Kennung aber, unterschrieben oder nicht, kann aber datentechnisch ohne weiteres entfernt werden, so dass die ursprüngliche Verordnung wieder herstellbar und somit einlösbar wäre. Damit wäre die bereits teilbelieferte Verordnungseinheit nochmals missbräuchlich einlösbar.

Die mit der Erfindung in Anspruch 2 erzielten Vorteile bestehen insbesondere darin, dass die Inhalte der elektronischen Verordnung durch den Patienten ohne Anwesendheit einer Signaturkarte eingesehen werden können und die Gültigkeit der Signatur überprüft werden kann.

Ein weiterer Vorteil für Anspruch 2 besteht darin, dass die elektronische Verordnung vor Missbrauch, speziell datentechnischem Duplizieren immanent geschützt ist. Denn ohne die separat abgelegte Informationseinheit, die zu je einer Verordnungseinheit assoziiert sind, kann die Verordnung weder teilbeliefert noch komplett beliefert werden. In Falle der Teilbelieferung wird nach Anspruch 1 für die Verordnungseinheit und nach Anspruch 2 für die zugehörige restliche Informationseinheit verfahren. Im Fall der kompletten Belieferung wird sowohl die elektronische Verordnung entsprechend der Speicherungsart von der Gesundheitskarte entfernt genauso wie die separate restliche Informationseinheit entfernt wird.

Die mit der Erfindung in Anspruch 3 erzielten Vorteile bestehen insbesondere darin, dass durch die Separierung in einzelne Verordnungseinheiten und Informationsblöcke aus Anspruch 1 die Folge der zusammengesetzten Hash-Werte sich mit der Zahl der Verordnungseinheiten und Informationsblöcke vergrößert. Durch Anwendung eines geeigneten Hash-Verfahren kann diese Folge verringert werden, so dass sich die relevante Prozessorzeit entsprechend verringert.

### Erweitere Ausgestaltung der Erfindung:

Weitere vorteilhafte Ergänzungen ergeben sich in Patentanspruch 4, indem das Gesamtdatenobjekt einer elektronischen Verordnung durch eine Objektkennung respektive einen Selbstidentifizierungscode erweitert werden kann. Dieses Datenobjekt kann Verfahrenszustände und elektronische Unterschriften zu den einzelnen Schritten beinhalten.

Weitere vorteilhafte Ergänzungen ergeben sich, indem die Werte einer Verordnungseinheit mit Status verordnet oder eines Informationsblocks sich aus mehreren unabhängigen Datenfeldern hinreichend zusammensetzen, was aber verfahrenstechnisch bekannt ist.

Nicht berücksichtigt ist die unvollständige Belieferung einer einzelnen Verordnungseinheit.

## Patentansprüche

1. Elektronische Verordnung (z.B. elektronisches Rezept)
• die auf ein Speichermedium (z.B. Gesundheitskarte) aufgebracht, geändert oder darüber referenziert wird oder ist,
• mit einem vorgegebenen elektronischen Signier- und Signaturprüfverfahren bestehend aus
- primärem Hash-Algorithmus und
- anschließender Verschlüsselung unter Einsatz einer Signaturkarte und
• mit den vorgegebenen vertraglichen Rahmenbedingungen insbesondere für Signatur, Datenschutz und des Anwendungsbereiches,
**dadurch gekennzeichnet,**
**dass** die elektronische Verordnung aus
• einer oder mehreren Verordnungseinheiten (z.B. Medikamente),
- die jeweils entweder im Status
"verordnet" (im Sprachgebrauch: verschrieben) oder
"beliefert" (im Sprachgebrauch: abgegeben oder eingelöst) vorliegen,
- die jeweils separat zeit- und ortsunabhängig voneinander beliefert werden können,
- im Status "verordnet" ist die Verordnungseinheit aus den notwendigen Teilinformationen (z.B. Pharmanummer) einer Einzelverordnung zusammengesetzt und
- im Status "beliefert" wird die Verordnungseinheit durch deren Hash-Wert ersetzt,
• einer oder mehreren Informationsblöcken
(z. B. restliche Rezeptinformationen inkl. Patientenidentifikation),
• einer elektronischen Signatur,
- die initial über allen Verordnungseinheiten und den Informationsblöcken erstellt wird,
- die für jede Verordnungseinheit und jeden Informationsblock den Hash-Wert separat ermittelt, soweit nicht die einzelne Einheit oder der Block durch den Hash-Wert bereits ersetzt ist, und
- die die zusammengesetzte Folge aus den ermittelten und ersetzten Hash-Werten für die Verschlüsselung bzw. die Signaturüberprüfung verwendet,
zusammengesetzt ist.

2. Elektronische Verordnung nach Anspruch 1
**dadurch gekennzeichnet,**
**dass** zu jeder Verordnungseinheit eine Informationseinheit,
• die separat von der elektronischen Verordnung abgelegt ist,
• deren Hash-Wert in der elektronischen Verordnung als ein Informationsblock gespeichert ist,
• die bei einer Teilbelieferung der zugehörigen "belieferten " Verordnungseinheit durch ihren Hash-Wert ersetzt wird,
• die bei Signaturprüfung der elektronischen Verordnung mit Signaturkarte herangezogen wird und
• deren Hash-Wert aus der elektronischen Verordnung bei Signaturprüfung ohne Signaturkarte herangezogen wird,
assoziiert ist.

3. Elektronische Verordnung nach Anspruch 1
**dadurch gekennzeichnet,**
**dass** elektronische Signatur aus der Hintereinanderanwendung von Hash-Verfahren und Verschlüsselung auf der geeignet angepassten und zusammengesetzten Folge aus den ermittelten und ersetzten Hash-Werten ermittelt wird.

4. Elektronische Verordnung nach Anspruch 1
**dadurch gekennzeichnet,**
**dass** die elektronische Verordnung um Zusatzinformation,
• die nicht in die elektronischen Signatur einfließt,
• die Verfahrensdaten und
• zu den Verfahrensdaten gehörige elektronische Signaturen enthält, ergänzt wird.
